# EUROPÄISCHE PATENTSCHRIFT

(11) **EP 2 059 582 B1**
(45) Veröffentlichungstag und Bekanntmachung des Hinweises auf die Patenterteilung: **08.05.2013**
(21) Anmeldenummer: 07820020.1
(22) Anmeldetag: 04.09.2007
(51) Int. Cl.: C11D 3/386

(54) **HOCHKONZENTRIERTES ENZYM-GRANULAT UND WASCH- ODER REINIGUNGSMITTEL, ENTHALTEND SOLCH EIN HOCHKONZENTRIERTES ENZYM-GRANULAT**
HIGH-CONCENTRATION ENZYME GRANULES AND DETERGENTS OR CLEANERS COMPRISING SUCH HIGH-CONCENTRATION ENZYME GRANULES
GRANULE D'ENZYME DE CONCENTRATION ELEVEE ET AGENT DE LAVAGE OU DE NETTOYAGE CONTENANT UN TEL GRANULE D'ENZYME DE CONCENTRATION ELEVEE

(30) Priorität: 08.09.2006 DE 102006042797; 26.06.2007 DE 102007029643
(43) Veröffentlichungstag der Anmeldung: 20.05.2009
(73) Patentinhaber: Henkel AG & Co. KGaA, 40589 Düsseldorf (DE)
(72) Erfinder: BESSLER, Cornelius, 40231 Düsseldorf (DE); KESSLER, Arnd, 40789 Monheim-Baumberg (DE); TONDERA, Susanne, 40597 Düsseldorf (DE); NITSCH, Christian, 40591 Düsseldorf (DE); ZIPFEL, Johannes, 40593 Düsseldorf (DE); MÜLLER-KIRSCHBAUM, Thomas, 42699 Solingen (DE); WARKOTSCH, Nadine, 40593 Düsseldorf (DE); MÜLLER, Sven, 47269 Duisburg (DE); HOLDERBAUM, Thomas, 40723 Hilden (DE)
(86) Internationale Anmeldenummer: PCT/EP2007/059203
(87) Internationale Veröffentlichungsnummer: WO 2008/028896

(56) Entgegenhaltungen:
- WO-A-00/63336
- WO-A-92/21760
- WO-A-97/49792
- WO-A-03/014358
- WO-A-2005/063974
- WO-A-2005/108537
- WO-A-2005/108540
- WO-A-2005/124012
- WO-A-2006/002643
- DE-A1- 10 309 803
- DE-A1- 10 360 841
- DE-A1- 19 651 446
- GB-A- 1 214 234
- GB-A- 1 300 596
- GB-A- 2 390 098
- US-A- 3 519 570
- US-A- 4 740 469
- US-A1- 2002 173 441

## Beschreibung

Die vorliegende Erfindung liegt auf dem Gebiet der Formulierung von Enzymgranulaten zur Einarbeitung in Wasch- und Reinigungsmittel. Sie betrifft ein maschinelles Geschirrspülmittel enthaltend ein hochkonzentriertes Enzym-Granulat sowie die Verwendung dieses Geschirrspülmittels.

Der Einsatz von Enzymen in Wasch- und Reinigungsmitteln ist seit Jahrzehnten im Stand der Technik etabliert. Sie dienen dazu, das Leistungsspektrum der betreffenden Mittel entsprechend ihren speziellen Aktivitäten zu erweitern. Hierzu gehören insbesondere hydrolytische Enzyme wie Proteasen, Amylasen, Lipasen und Cellulasen. Die ersten drei genannten hydrolysieren Proteine, Stärke und Fette und tragen somit unmittelbar zur Schmutzentfemung bei. Cellulasen werden insbesondere wegen ihrer Gewebewirkung eingesetzt. Eine weitere Gruppe von Wasch- und Reinigungsmittelenzymen sind oxidative Enzyme, insbesondere Oxidasen, die ggf. im Zusammenspiel mit anderen Komponenten vorzugsweise dazu dienen, Anschmutzungen zu bleichen. Enzyme wie beispielsweise Perhydrolasen dienen dazu, die bleichenden Agentien *in situ* zu erzeugen. Neben diesen Enzymen werden laufend weitere Enzyme für den Einsatz in Wasch- und Reinigungsmitteln bereitgestellt, insbesondere um spezielle Anschmutzungen optimal angehen zu können, wie beispielsweise Pektinasen, β-Glucanasen, Mannanasen oder weitere Hemicellulasen zur Hydrolyse insbesondere spezieller pflanzlicher Polymere.

Die am längsten etablierten und in praktisch allen modernen, leistungsfähigen Wasch- und Reinigungsmitteln enthaltenen Enzyme sind Proteasen und hierunter insbesondere Serin-Proteasen, zu denen erfindungsgemäß auch die Subtilasen gerechnet werden. Sie dienen dem Abbau proteinhaltiger Anschmutzungen auf dem Reinigungsgut. Unter den Wasch- und Reinigungsmittelproteasen nehmen Subtilasen aufgrund ihrer günstigen enzymatischen Eigenschaften wie Stabilität oder pH-Optimum eine herausragende Stellung ein. Daneben sind auch andere Protease-Typen für diesen Zweck beschrieben, beispielsweise Metalloproteasen.

Subtilasen (Proteasen vom Subtilisin-Typ, Subtilopeptidasen, EC 3.4.21.62) wirken als unspezifische Endopeptidasen, das heißt, sie hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisinlike Proteases" von R.Siezen, Seite 75-95 in "Subtilisin enzymes", herausgegeben von R.Bott und C.Betzel, New York, 1996. Subtilasen werden natürlicherweise von Mikroorganismen, gebildet; hierunter sind insbesondere die von *Bacillus*-Spezies gebildeten und sekretierten Subtilisine als bedeutendste Gruppe innerhalb der Subtilasen zu erwähnen.

Die wichtigsten Vertreter hiervon sind die Alkalischen Proteasen aus *Bacillus amyloliquefaciens* (BPN') und *Bacillus licheniformis* (Subtilisin Carlsberg), die Alkalische Protease PB92, Subtilisin 147 und/oder 309 (die unter dem Handelsnamen Savinase von der Fa. Novozymes A/S, Bagsværd, Dänemark, erhältlich ist) sowie die Alkalische Protease aus *Bacillus lentus,* vor allem aus *Bacillus lentus* (DSM 5483) und jeweils die über Mutagenese erhältlichen Varianten dieser Enzyme.

Als nächstwichtige Enzymklasse sei auf Amylasen verwiesen, unter denen α-Amylasen eine herausragende Stellung einnehmen, zu denen aber auch andere amylolytische Enzyme wie etwa Cyclodextrin-Glucanotransferasen (CGTasen) gerechnet werden.

α-Amylasen (E.C. 3.2.1.1) hydrolysieren interne α-1,4-glycosidische Bindungen von Stärke und stärkeähnlichen Polymeren. Weil Wasch- oder Reinigungsmittel überwiegend alkalische pH-Werte aufweisen, werden hierfür insbesondere α-Amylasen eingesetzt, die im alkalischen Medium aktiv sind. Solche werden von Mikroorganismen, das heißt Pilzen oder Bakterien, vor allem denen der Gattungen *Aspergillus* und *Bacillus* produziert und sekretiert. Ausgehend von diesen natürlichen Enzymen steht inzwischen eine nahezu unüberschaubare Fülle von Varianten zur Verfügung, die über Mutagenese abgeleitet worden sind und je nach Einsatzgebiet spezifische Vorteile aufweisen.

Beispiele hierfür sind die α-Amylasen aus *Bacillus licheniformis* (von der Firma Novozymes unter dem Namen Termamyl® und von der Firma Genencor unter dem Namen Purastar®ST erhältlich), aus *B*. *amyloliquefaciens* (von der Firma Novozymes unter dem Namen BAN® vertrieben) und aus *B. stearothermophilus* (von der Firma Novozymes unter dem Namen Novamyl® vertrieben) sowie deren für den Einsatz in Wasch- oder Reinigungsmitteln verbesserte Weiterentwicklungen.

Auch zu dem anderen oben erwähnten hydrolytischen Enzym besteht ein umfangreicher Stand der Technik, insbesondere zu den Lipasen und Cellulasen, die ebenfalls seit langem in Wasch- und Reinigungsmitteln eingesetzt werden.

Bezüglich des Einsatzes oxidativer Enzyme sei beispielhaft auf die Anmeldung WO 97/21796 A1 verwiesen, woraus der Einsatz von Alkalischen Oxidasen, insbesondere Cholin-Oxidasen hervorgeht. Hierfür geeignete Enzyme offenbart beispielsweise WO 2004/058955 A2. Ein Beispiel für die enzymatische Perhydrolase liefert WO 98/45398 A1. Ein neues enzymatisches Bleichsystem, umfassend mindestens eine Oxidase und mindestens eine Perhydrolase, offenbart schließlich WO 2005/124012 A1.

Es wird beständig daran gearbeitet, für das Technikgebiet der Wasch- und Reinigungsmittel neue und besser geeignete Enzyme zur Verfügung zu stellen, sowohl innerhalb der oben genannten Enzymklassen als auch aus Enzymklassen, die bislang noch nicht für dieses Einsatzgebiet beschrieben worden sind. Ein Weg, zu neuen Enzymen, insbesondere zu neuen Vertretern innerhalb der etablierten Klassen zu gelangen, besteht darin, über mikrobiologische Methoden natürliche Habitate zu durchmustern (Screening) und Mikroorganismen daraus zu isolieren, die die betreffenden Enzyme bilden. Dieser Ansatz ist inzwischen dahingehend erweitert worden, dass beispielsweise Protease-codierende DNA direkt aus Proben natürlichen Ursprungs gewonnen wird. Beispielhaft sei hierfür auf die Anmeldungen WO 2005/063974 A1 und WO 2005/103244 A1 verwiesen.

Natürliche (Wildtyp-)Enzyme werden über an sich bekannte Mutagenese-Verfahren beispielsweise für den Einsatz in Wasch- und Reinigungsmitteln optimiert. Dies geschieht in der Absicht, beispielsweise Leistungsparameter, Wechselwirkungen mit dem Substrat, die Thermostabilität oder die Stabilität gegenüber denaturierenden oder inaktivierenden Agentien zu verbessern, wie z.B. gegenüber Tensiden bzw. Bleichmitteln. Werden gezielte Sequenzänderungen vorgenommen, spricht man von Rationalem Protein-Design.

Eine moderne Richtung der Enzymentwicklung besteht darin, Elemente aus bekannten, miteinander verwandten Enzymen über statistische Verfahren zu neuen Enzymen mit bislang nicht erreichten Eigenschaften zu kombinieren. Solche Verfahren werden auch unter dem Oberbegriff Directed Evolution zusammengefasst. Dazu gehören beispielsweise folgende Verfahren: Die StEP-Methode (Zhao et al. (1998), Nat. Biotechnol., Band 16, S. 258-261), Random priming recombination (Shao et al., (1998), Nucleic Acids Res., Band 26, S. 681-683), DNA-Shuffüng (Stemmer, W.P.C. (1994), Nature, Band 370, S. 389-391) oder RACHITT (Coco, W.M. et al. (2001), Nat. Biotechnol., Band 19, S. 354-359). Eine weitere Shuffling-Methode mit der Bezeichnung "Recombining ligation reaction" (RLR) ist in WO 00/09679 A1 beschrieben.

Wie all diese über einen langen Zeitraum durchgeführten Arbeiten belegen, besteht ein hoher Bedarf an Enzymen, die sich für den Einsatz in Wasch- und Reinigungsmitteln eignen. Insbesondere der Leistungsaspekt ist dabei von Interesse. Denn je höher die Leistung eines Enzyms ist, desto besser ist die vom Endverbraucher geschätzte Reinigungswirkung. Umgekehrt können mit einer leistungsfähigeren Enzymkomponente dieselben guten Ergebnisse bereits mit geringeren Konzentrationen erzielt werden, was die Konzeptionierung der betreffenden Rezepturen erleichtert, Raum für andere Inhaltsstoffe schafft und/oder die mit der Enzymkomponente verbundenen Kosten senkt.

Ein anderer Gesichtspunkt zum Einsatz von Enzymen in Wasch- und Reinigungsmitteln ist deren Konfektionierung, d.h. der physikalischen Form, in der sie den betreffenden Mitteln zugesetzt werden können.

Dazu, und insbesondere zur Konfektionierung in fester Form existiert ebenfalls ein umfangreicher Stand der Technik. Hierzu gehören Partikel oder besser, weil aus mehreren Inhaltsstoffen bestehend: Granulatkörner (Granula, Granalien), die in ihrer Summe die Konfektionierungsform des Granulats ergeben. Zur Herstellung von Wasch- und Reinigungsmitteln ist es üblich, verschiedenste Inhaltsstoffe und so eben auch Enzyme in Form von Granulaten in entsprechende, zumeist feste Mittel einzuarbeiten.

Eine grundsätzliche Alternative hierzu stellt die Konfektionierung von Enzymen in flüssiger Form dar, welche in der Regel in überwiegend flüssige oder gelförmige Wasch- und Reinigungsmittel eingearbeitet werden. Allerdings sind sie in dieser Form nicht physikalisch gegen negative Einflüsse anderer Inhaltsstoffe der betreffenden Wasch- und Reinigungsmittel geschützt, so dass Enzyme auch flüssigen Mitteln vorzugsweise in Form fester Granulate zugesetzt werden. Neuere Entwicklungen zielen darauf ab, die insbesondere physikalische Stabilität derartiger Granulate und/oder die Aktivität der in den Granulaten enthaltenen Enzyme, insbesondere bei Einarbeitung in ein überwiegend flüssiges Wasch- oder Reinigungsmittel durch geeignete Schutzmaßnahmen dauerhaft zu erhalten.

Die Enzyme flüssigen Mitteln in fester Form zuzusetzen, wird von WO 99/00471 A1 und WO 99/00478 A1 offenbart. In beiden Fällen werden die Enzyme zu so genannten Prills konfektioniert, wie sie auch für den Einsatz in festen Mitteln üblich sind. Die erste dieser Anmeldungen vermittelt hierbei die Lehre, diese festen Partikel hinsichtlich ihrer Dichte an die des Mediums anzugleichen, um während der Lagerung eine hinreichende Stabilität der Partikel zu gewährleisten. Die zweite dieser Anmeldungen vermittelt die Lehre, die Reinigungsleistung der enthaltenen Enzyme durch Zugabe der Verbindung Ethylendiamin-N,N'-Disuccinsäure (EDDS) oder deren Salze zu erhöhen.

Andere Entwicklungen zielten schwerpunktmäßig darauf ab, die Enzymgranula oder Partikel mit anderen Inhaltsstoffen zusätzlich mit einer Schutzschicht (Coating) zu überziehen. Beispielsweise WO 00/29534 A1 offenbart die Herstellung von Granulaten, bei denen verschiedene Schichten auf einen inerten Kern oder Träger aufgebracht werden. Hierunter kann auch eine Enzymschicht sein, die obligatorisch nach außen durch eine oder mehrere Schutzschichten überdeckt wird.

Eine ganze Reihe von Anmeldungen offenbart verschiedene Beschichtungs-Materialien oder Kombinationen von Materialien oder verschiedene Methoden, sie zu applizieren, z.B. als Puder, als Lösung oder als Schmelze. Die Beschichtungen erfüllen dabei jeweils einen doppelten Zweck: (1.) physikalische und chemische Stabilität gegenüber dem Einfluss von anderen Wasch- bzw. Reinigungsmittelinhaltsstoffen während der Lagerung und (2.) rasche oder regulierte Freisetzung der Inhaltsstoffe im Augenblick des Einsatzes des Mittels, d.h. der Verdünnung mit Wasser zu einer Wasch- bzw. Reinigungsflotte.

Eine beide Zwecke erfüllende Konfektionierungsmethode geht beispielsweise aus der nicht vorveröffentlichten Anmeldung DE 102006018780.6 hervor. Sie offenbart Granulate sensitiver Wasch- oder Reinigungsmittelinhaltsstoffe, enthaltend die Komponenten: (a) den sensitiven Wasch- oder Reinigungsmittelinhaltsstoff, d.h. beispielsweise das Enzym, (b) ein partikuläres Trägermaterial (Adsorbens), (c) einen von (b) unterschiedlichen Inhaltsstoff als Bindemittel und (d) optional weitere, von (b) und (c) unterschiedliche Inhaltsstoffe, welche bei Lagerung bei 23°C in einem wässrigen Puffersystem, bestehend aus 16% Natriumsulfat und 3% Natriumcitrat, pH 5,0 ± 0,1 über einen Zeitraum von 24 h im wesentlichen nicht desintegrieren.

Ob jedoch über die Art der Granulate und insbesondere die Konzentration der enthaltenen Enzyme ein Einfluss auf die Wasch- bzw. Reinigungsleistung der enthaltenen Enzyme genommen werden kann, ist bislang noch nicht in Erwägung gezogen worden. Bislang war es, abgesehen von der Auswahl des Enzyms an sich, nur möglich, die auf das Enzym zurückzuführende Leistung lediglich über die Gesamtmenge der Enzymkonzentration, d.h. durch die Einwaage der zur Verfügung stehenden Granulate zu beeinflussen.

Es stellte sich somit die Aufgabe, die Wasch- bzw. Reinigungsleistung von enzymhaltigen Wasch- oder Reinigungsmitteln durch die Entwicklung einer leistungsfähigeren Enzymkomponente zu verbessern. Es sollten also leistungsverbesserte Enzymkomponenten, auf diese Weise leistungsverbesserte Wasch- und/oder Reinigungsmittel sowie entsprechende Wasch- und Reinigungsverfahren und -Verwendungsmöglichkeiten zur Verfügung gestellt werden.

Hierbei war es nicht das vorrangige Interesse, ein an sich neues Enzym zu entwickeln, sondern eine Leistungsverbesserung zu entwickeln, die grundsätzlich auf alle Enzyme, insbesondere auf Proteasen und Amylasen anwendbar ist.

Diese Aufgabe wird gelöst durch ein phosphatfreies maschinelles Geschirrspülmittel, enthaltend bezogen auf das Gesamtgewicht des maschinellen Geschirrspülmittels,
a) 0,2 bis 7 Gew.-% Enzymgranulat mit einem Enzymgehalt von 5,5 bis 30 Gew.-% an Aktivsubstanz des Enzyms, wobei es sich bei dem Enzym um ein Subtilisin handelt, bei welchem es sich um ein Wildtypenzym oder um eine Subtilisin-Variante handelt, wobei es sich bei dem Wildtypenzym bzw. dem Ausgangsenzym der Variante um die alkalische Protease aus *Bacillus amyloliquefaciens* (BPN') handelt und wobei es sich bei dem Enzym um eine Variante handelt, die zu der Ausgangsprotease auf Aminosäureebene über die Gesamtlänge der Variante eine Sequenzidentität von mindestens 90 % aufweist;
b) zwischen 5 bis 60 Gew.-% Citrat/Citronensäure;
c) zwischen 10 bis 60 Gew.-% Carbonat.

Neben dem zuvor bezeichneten phosphatfreien maschinellen Geschirrspülmittel ist weiterer Gegenstand der vorliegenden Erfindung die Verwendung eines erfindungsgemäßen maschinellen Geschirrspülmittels zur Beseitigung Eiweiß-haltiger Verschmutzungen in einem maschinellen Geschirrspülverfahren.

Weiterer Gegenstand der vorliegenden Erfindung ist ebenso ein Verfahren zur Reinigung von Geschirr in einer Geschirrspülmaschine unter Einsatz eines erfindungsgemäßen maschinellen Geschirrspülmittels.

Die mit dieser Erfindung zu erzielenden Vorteile werden durch Beispiel 1 der vorliegenden Anmeldung illustriert, ohne dass die Erfindung und ihre Ausführungsformen darauf eingeschränkt wären. Dort ist belegt, dass bei Einsatz eines erfindungsgemäß konzentrierten Enzymgranulats im Rahmen einer Geschirrspülmittelrezeptur eine bessere Reinigungsleistung erzielt werden kann, als bei niedriger konzentrierten Granulaten. Dieser Effekt ist überraschenderweise von der in dem betreffenden Mittel eingestellten Endkonzentration des betreffenden Enzyms unabhängig. Umgekehrt kann in entsprechenden Mitteln dieselbe Leistung mit einer geringeren Absolutmenge an Enzym erzielt werden, wodurch eine Substanzerspamis erreicht werden kann und bei vorgegebenem Volumen eines solchen Mittels mehr Volumen für andere Wirksubstanzen zu Verfügung steht.

Anwendungsgebiete der vorliegenden Erfindung umfassen alle denkbaren und/oder zweckmäßigen Darreichungsformen der erfindungsgemäßen maschinellen Geschirrspülmittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Hierfür besonders geeignete Inhaltsstoffe und Konfektionierungsmöglichkeiten werden weiter unten ausführlich dargestellt.

Unter einem Enzymgranulat wird erfindungsgemäß jede feste Konfektionierungsform von Enzymen verstanden, beispielsweise Prills, Granulate, Extrudate oder durch Vertropfung erhältliche Partikel, jeweils in unbeschichteter oder ein- oder mehrfach beschichteter Form.

Derartige Enzymgranulate eignen sich erfindungsgemäß dann für die Einarbeitung in Wasch- und/oder Reinigungsmittel, wenn sie bei der Herstellung der betreffenden, enzymgranulathaltigen Mittel und deren anschließender Lagerung im wesentlichen nicht desintegrieren und ihre Enzymaktivität so weit behalten, dass die betreffende Enzymaktivität zum Zeitpunkt des Einsatzes noch so hoch ist, dass von dem betreffenden Enzym als von einer Wirksubstanz des betreffenden Mittels gesprochen werden kann.

Da je nach Herstellverfahren von Granulaten die betreffenden Inhaltsstoffe mit Zuschlagstoffen beaufschlagt werden, ist jeweils der Gehalt an Aktivsubstanz eine entscheidende Größe. Hierunter ist der Anteil an reinem, aktiven Enzymprotein zu verstehen. Dieser wird bei der Herstellung der Granulate eingestellt. Gleichzeitig kann eine Bestimmung der betreffenden Enzymaktivität vorgenommen werden. Diese steht i.d.R. in einem festen, jeweils enzymspezifischen Verhältnis zur Aktivsubstanz. Während langer Lagerung der Granulate nimmt die Enzymaktivität aufgrund von Umwelteinflüssen ab; dementsprechend sinkt auch der Gehalt an Aktivsubstanz. Es ist somit möglich, beispielsweise über Titration mit einem Inhibitor des betreffenden Enzyms zu jedem beliebigen Zeitpunkt die Enzymaktivität zu messen und hierüber den Gehalt an Aktivsubstanz zu ermitteln.

So wurden in Beispiel 1 zur vorliegenden Anmeldung zwei verschiedene Granulate einer Alkalischen Protease hergestellt, und zwar mit einem Aktivsubstanzgehalt der Protease von 6,9 Gew.-% und von 4,5 Gew.-% im jeweiligen Granulat. Zu deren Herstellung war eine Präparation dieser Protease mit 5.797 Protease-Einheiten (PE) pro mg eingesetzt worden (bestimmt nach Tenside, Band 7 (1970), Seite 125-132). Die Einwaage von 69 mg bzw. 45 mg auf 1 g Granulat lieferte die genannten 6,9 bzw. 4,5 Gew.-% Aktivsubstanz und Granulate mit ca. 400.000 bzw. ca. 260.869 PE pro g Granulat.

Bevorzugt eingesetzte Proteasegranulate basieren dementsprechend auf solchen Proteasepräparationen, die 3.000 bis 9.000, vorzugsweise 4.000 bis 8.000, besonders bevorzugt 5.000 bis 7.000, ganz besonders bevorzugt 5.500 bis 6.500 Protease-Einheiten pro mg besitzen. Hierdurch werden beispielsweise bei einem Aktivsubstanzgehalt von 10 Gew.-% im Granulat erfindungsgemäße Proteasegranulate mit 300.000 bis 900.000, 400.000 bis 800.000, 500.000 bis 700.000 bzw. 550.000 bis 650.000 Protease-Einheiten pro g Granulat erhalten.

Erfindungsgemäß eingesetzte Enzymgranulate enthalten 5,5 bis 30 Gew.-% an Aktivsubstanz des Enzyms. In einer bevorzugten Ausführungsform hat das Enzymgranulat einen Enzymgehalt von zunehmend bevorzugt 6,2 bis 30 Gew.-%, 6,4 bis 20 Gew.-%, 6,6 bis 17,5 Gew.-%, 6,7 bis 15 Gew.-%, ganz besonders bevorzugt 6,8 bis 12 Gew.-% Aktivsubstanz.

Wie in Beispiel 1 beschrieben wird mit Proteasegranulaten, die einen Gehalt von 6,9 Gew.-% an Aktivsubstanz in einer Reinigungsmittelrezeptur eine bessere Waschleistung erzielt als mit solchen, deren Gehalt an Aktivsubstanz unter 5 Gew.-% liegt. Hierbei handelt es sich um den überraschenden, erfindungsgemäßen Effekt.

Dieser kann bei Einhaltung der Konzentrationsangabe für das Enzym grundsätzlich mit allen Arten von Enzymgranulaten erzielt werden, ungeachtet deren sonstiger Konzeptionierung. So enthalten zahlreiche Granulate inerte Begleitstoffe als Füllsubstanzen, insbesondere um die Herstellung der Granulate, zu ermöglichen und deren Lagerstabilität in entsprechenden Mitteln zu gewährleisten. Weitere übliche Inhaltsstoffe in erfindungsgemäßen Granulaten sind beispielsweise:
- Füllstoffe mit niedriger Dichte, um für die Granulate ein definiertes, vergleichsweise niedriges spezifisches Gewicht einzustellen;
- Wasser oder andere Flüssigkeiten zur Beeinflussung der Viskosität des zu verarbeitenden Materials und der physikalischen Eigenschaften des Granulats;
- Farbstoffe, beispielsweise Pigmente;
- Wirksubstanzen, die dem beabsichtigten Einsatzgebiet der betreffenden Granulate in Wasch- und/oder Reinigungsmitteln zugute kommen, beispielsweise mit den Enzymen verträgliche Tenside;
- Stabilisatoren zur Erhöhung der Enzymstabilität.

Subtilisin BPN', welches natürlicherweise aus *Bacillus amyloliquefaciens,* beziehungsweise B. *subtilis* stammt, ist aus den Arbeiten von Vasantha et al. (1984) in J. Bacteriol., Volume 159, S. 811-819 und von J. A. Wells et al. (1983) in Nucleic Acids Research, Volume 11, S. 7911-7925 bekannt. Subtilisin BPN' dient insbesondere hinsichtlich der Numerierung der Positionen als Referenzenzym der Subtilisine. So werden beispielsweise die auf alle Subtilisine bezogenen Punktmutationen der Anmeldung EP 251446 A1 in der Numerierung von BPN' angegeben, deren Anmeldungsgegenstand von der Fa. Procter & Gamble Comp., Cincinnati, Ohio, USA, als "Protease B" bezeichnet wird.

Die BPN'-Varianten der Anmeldung EP 199404 A1 werden von Procter & Gamble Comp. als "Protease A" bezeichnet. "Proteasen C" zeichnen sich gemäß der Anmeldung WO 91/06637 A1 durch wiederum andere Punktmutationen von BPN' aus. Bei der "Protease D" handelt es sich gemäß WO 95/10591 A1 um Varianten der Protease aus *Bacillus lentus* (s.u.).

WO9505440 beschreibt ein phosphatfreies maschinelles Geschirrspülmittel, ausgewählt aus Granulaten oder Puder, und enthält 0 bis 8 Gew.-% , bzw. 0,001-5 Gew. -% , bzw.0.003- 4 Gew % Enzyme , und zwischen 10-30 Gew.-% , Citrat , 7-25 Gew.-% Carbonat, 11,4 Gew.-%, Natriumpercarbonat und bis 10 Gew.-% nicht ionische Tensid.WO9505440 enthält Amineoxide mit ungesättigten Carbonsäuren. Die Enzymen werden ausgewählt aus Protease, Amylase und Lipase.

In einer bevorzugten Ausführungsform wird ein Enzymgranulat eingesetzt, wobei es sich bei der Protease um eine Variante handelt, die zu der Ausgangsprotease auf Aminosäureebene über die Gesamtlänge der Variante eine Sequenzidentität von mindestens 90%, vorzugsweise mindestens 92,5%, besonders bevorzugt mindestens 95% und ganz besonders bevorzugt mindestens 97,5% aufweist, unabhängig davon, ob sie von dieser selbst oder von einem anderen Enzym abgeleitet worden ist.

In einer ebenfalls bevorzugten Ausführungsform wird ein Enzymgranulat eingesetzt, wobei es sich bei der Protease-Variante um eine Variante mit einer Punktmutation in Position 217 (Zählung gemäß der Wildtyp-Protease aus *Bacillus amyloliquefaciens;* BPN') handelt, vorzugsweise mit einer Substitution einer einzelnen Aminosäure in dieser Position, besonders bevorzugt mit der Aminosäuresubstitution X217L, ausgehend von der Wildtyp-Protease aus *Bacillus amyloliquefaciens* (BPN'), bzw. einer Variante davon.

Bei der Position 217 in der Zählung gemäß der Wildtyp-Protease aus *Bacillus amyloliquefaciens* (BPN') handelt es sich um dieselbe Position, die in der Zählung nach *Bacillus lentus*-Alkalische Protease oder Savinase® als Position 211 bezeichnet werden muss. Über leistungsverbessernde Mutationen in dieser Position gibt es zahlreiche Beschreibungen, beispielsweise für die *Bacillus lentus*-Alkalische Protease in WO 95/23221 A1. Dort werden die Substitutionen L211 D und L211E gegenüber dieser Protease offenbart.

Der erfindungsgemäß besonders bevorzugte Austausch einer beliebigen Aminosäure (X) gegen ein Leucin (L) in dieser Position geht aus US 6908757 B1 hervor. Er ist erfindungsgemäß besonders dann bevorzugt, wenn von der Wildtyp-Protease aus *Bacillus amyloliquefaciens* (BPN'), bzw. einer Variante davon ausgegangen wird. Derartige Proteasen sind beispielsweise unter dem Handelsnamen Purafect Prime® von der Fa. Genencor erhältlich.

In einer bevorzugten Ausführungsform hat das Enzymgranulat eine Korngröße von 0.1 mm bis 2 mm und enthält 2 Gew.-% bis 20 Gew.-% Protease, berechnet als Trockensubstanz, 10 Gew.-% bis 50 Gew.-% quellfähige Stärke, 5 Gew.-% bis 50 Gew.-% Granulierhilfsmittel, das ein wasserlösliches organisches Polymer enthält, nicht über 10 Gew.-% wasserlösliches Salz und 3 Gew.-% bis 12 Gew.-% Wasser, welches 10 Gew.-% bis 35 Gew.-% Getreidemehl enthält.

Die Herstellung solcher Granulate ist aufgrund der Anmeldung WO 92/11347 A1 möglich. Daraus geht hervor, dass die zur Enzymkomponente hinzukommende Inhaltsstoffkombination auch bei Einsatz niedrigkonzentrierter Fermentationsbrühen eine gute Lagerstabilität und schnelle Freisetzung der Inhaltsstoffe gewährleistet.

In einer bevorzugten Ausführungsform enthält das Enzymgranulat Enzym und anorganisches und/oder organisches Trägermaterial sowie Granulierhilfsmittel, wobei es als Granulierhilfsmittel eine phosphatierte Stärke enthält.

Die Herstellung solcher Granulate ist aufgrund der Anmeldung WO 97/40128 A1 möglich. Daraus geht hervor, dass die Zugabe gerade dieses Granulierhilfsmittels die Zerfallsgeschwindigkeit der damit herstellbaren Enzymgranulate erhöht und deren Einspülbarkeit in übliche Waschmaschinen verbessert.

In einer bevorzugten Ausführungsform enthält das Enzymgranulat natürlicherweise von den zur Fermentation eingesetzten Mikroorganismen gebildete stabilisierende Begleitstoffe.

Dies kann, wie in der Anmeldung WO 2005/063975 A1 beschrieben ist, dadurch erreicht werden, dass in die Enzymgranulate Enzymkonzentrate eingearbeitet werden, die chromatographisch, d.h. ohne zwischenzeitliche Fällung und somit schonend entfärbt und desodoriert worden sind. Dies geschieht vorteilhafterweise, indem von einer wässrigen, gereinigten Enzymlösung ausgegangen wird, die (a) einer lonenaustausch-Chromatographie unterworfen wird, während der das interessierende Enzym in Lösung bleibt, (b) anschließend eine geeignete Konzentration eingestellt wird und dann in Schritt (c) die Granulation nach an sich bekannten Methoden erfolgt.

In einer bevorzugten Ausführungsform ist das Enzymgranulat dadurch gekennzeichnet, dass die Granulatpartikel hygroskopische Polyole enthalten.

Die Herstellung solcher Granulate ist aufgrund der Anmeldung WO 2005/108539 A1 möglich. Daraus geht hervor, dass derartige feste Granulate eine ausgezeichnete Lagerstabilität und Abriebfestigkeit aufweisen. In bevorzugten Ausführungsformen werden diese Granulate durch Extrusion hergestellt. Bevorzugte hygroskopische Polyole sind ausgewählt unter Ethylengykol, Propylenglykol, Triethylenglykol, Glycerin, Monoglyceriden, Diglyceriden, Polyethylenglykolen (PEG), Polypropylenglykolen (PPG), Polyvinylalkoholen (PVA), Polysacchariden, Celluloseethern, Alginaten, modifizierten Stärken und deren Hydrolysaten, den Polymeren und Copolymeren.dieser Verbindungen oder deren Copolymeren mit anderen Polymeren, welche unter Polyethylenoxiden, Polyvinylpyrrolidonen (PVP) und Gelatine ausgewählt sind, insbesondere ausgewählt unter: Glycerin, Cellulose, Sorbit, Saccharose und Stärke. Vorteilhafterweise werden die hygroskopischen Polyole in einer Menge von 0,1 bis 10 Gew.-%, insbesondere von 3 bis 7 Gew.-% einsetzt.

In einer bevorzugten Ausführungsform enthält das Enzymgranulat die Komponenten:
(a) das Enzym,
(b) ein partikuläres Trägermaterial (Adsorbens),
(c) einen von (b) unterschiedlichen Inhaltsstoff als Bindemittel und
(d) optional weitere, von (b) und (c) unterschiedliche Inhaltsstoffe,
welches bei Lagerung bei 23°C in einem wässrigen Puffersystem, bestehend aus 16% Natriumsulfat und 3% Natriumcitrat in Wasser, pH 5,0 ± 0,1 über einen Zeitraum von 24 h im wesentlichen nicht desintegriert.

Die Herstellung solcher Granulate ist aufgrund der nicht vorveröffentlichten Anmeldung DE 102006018780.6 möglich. Daraus geht hervor, dass gerade diese Zusammensetzung diesen Granulaten eine ausreichende Stabilität für die Lagerung in flüssigen Rezepturen verleiht, auch wenn sie nicht zusätzlich beschichtet sind. Gleichwohl kann eine zusätzliche Beschichtung vorteilhaft sein (s.u.).

In bevorzugten Ausführungsformen hiervon weisen die Komponenten (b) und (c) ein Gew.-%-Verhältnis von (b) zu (c) von 1 : 50 zu 50 : 1, vorzugsweise von 1 : 20 zu 20 : 1, besonders bevorzugt von 1 : 5 zu 5 : 1 auf. Weiterhin bevorzugt sind für die genannten Komponenten folgende Mengenbereiche:
(a) das Enzym,
(b) 10 - 80 Gew.-% eines partikulären Trägermaterials (Adsorbens),
(c) 3 - 50 Gew.-% eines von (b) unterschiedlichen Inhaltsstoffs als Bindemittel und
(d) als optionale weitere, von (b) und (c) unterschiedliche Inhaltsstoffe:
   0 - 50 Gew.-% (bezogen auf das Granulat) Plastifizierer,
   0 - 50 Gew.-% (bezogen auf das Granulat) Löslichkeitsverbesserer (Quellmittel, Desintegrationshilfsmittel, Sprengmittel), und/oder
   0 - 40 Gew.-% (bezogen auf das Granulat) Wasser, Enzymstabilisatoren, Farbstoffe, Farbpigmente, pH-Puffersubstanzen, Antioxidantien, die Dichte regulierende Verbindungen und/oder weitere Inhaltsstoffe.

Bevorzugte Komponenten (b) (Adsorbens) sind eine oder mehrere der Verbindungen, ausgewählt aus der Gruppe: Talkum, Kieselsäure, Aluminiumoxid, Silikat, insbesondere Schichtsilikat und/oder Natriumaluminiumsilikat, Bentonit, Alumosilikat (Zeolith), Sulfat, Titandioxid und/oder Polyvinylalkohol (PVA), insbesondere teilhydrolysierten PVA, besonders bevorzugt eine Kombination von zweien oder dreien dieser Verbindungen.

Bevorzugte Komponenten (c) (Bindemittel) sind eine oder mehrere der Verbindungen, ausgewählt aus der Gruppe: Polyacrylat, Polymethacrylat, Polyvinylpyrrolidon, Polysaccharid oder substituiertes Polysaccharid, insbesondere Celluloseether, Polyvinylalkohol (PVA), insbesondere teilhydrolysierten PVA oder ethoxylierten PVA, ein Copolymer der genannten Verbindungen, insbesondere Methacrylsäure-Ethylacrylat-Copolymer, besonders bevorzugt eine Kombination von zweien oder dreien dieser Verbindungen.

Bevorzugt als Teil der Komponente (d) (Plastifizierer) sind eine oder mehrere Verbindungen, ausgewählt aus der Gruppe: wasserdispergierbare organische Verbindung oder wasserdispergierbares organisches Polymer, insbesondere Polyethylenglykol (PEG), ganz besonders kurzkettiges PEG, Fettsäure oder Salz einer Fettsäure, Triacetin, Triethylcitrat und/oder mehrwertigen Alkohol, vorzugsweise Fettsäure oder Salz einer Fettsäure, besonders bevorzugt Natriumstearat und/oder Natriumoleat.

Bevorzugt als Teil der Komponente (d) (Löslichkeitsverbesserer) sind eine oder mehrere Verbindungen, ausgewählt aus der Gruppe: wasserlösliches anorganisches Salz, Monosaccharid, insbesondere Glucose, Oligosaccharid, Polysaccharid, insbesondere Cellulose, kompaktierte Cellulose oder Cellulosederivat, quervernetztes organisches Polymer, insbesondere quervernetztes Polyvinylpyrrolidon oder quervernetztes Polyacrylat.

Eine bevorzugte Ausführungsform ist ein erfindungsgemäßes Enzymgranulat, welches eine Beschichtung (Coating) aufweist.

Ausführungsformen der vorliegenden Erfindung umfassen wie ebenfalls bereits gesagt alle denkbaren und/oder zweckmäßigen Darreichungsformen der erfindungsgemäßen Geschirrspülmittel.

Erfindungsgemäß einsetzbare Enzymgranulate werden in erfindungsgemäßen Mitteln beispielsweise mit einzelnen oder mehreren der folgenden Inhaltsstoffe kombiniert: nichtionische, anionische, kationische oder amphotere Tenside, Bleichmittel, Bleichaktivatoren, Bleichkatalysatoren, Builder und/oder Cobuilder, Lösungsmittel, Verdicker, Sequestrierungsmittel, Elektrolyte, optische Aufheller, Vergrauungsinhibitoren, Korrosionsinhibitoren, insbesondere Silberschutzmittel, Soil-Release-Wirkstoffe, Farbtransfer(oder -Übertragungs)-Inhibitoren, Schauminhibitoren, Abrasivstoffe, Farbstoffe, Duftstoffe, antimikrobielle Wirkstoffe, UV-Schutzmittel, weitere (gegebenenfalls ebenfalls erfindungsgemäß granulierte) Enzyme wie beispielsweise Proteasen, Amylasen, Lipasen, Cellulasen, Hemicellulasen oder Oxidasen, Stabilisatoren, insbesondere Enzymstabilisatoren, und andere Komponenten, die aus dem Stand der Technik bekannt sind.

Überraschenderweise wurde gefunden, dass sich besonders vorteilhafte Eigenschaften der Mittel ergeben, wenn die Proteasegranulate mit speziellen Inhaltsstoffen kombiniert werden. Hierunter sind folgende Kombinationen hervorzuheben:
- Kombination mit Carboxymethylcellulose zur Verbesserung der Soil-Release-Eigenschaften;
- Kombination mit Bleichmitteln für eine verbesserte Schmutzentfernung;
- Kombination mit anderen Proteasen für eine verbesserte Leistung der betreffenden Mitteln an den Protease-sensitiven Anschmutzungen Ei, Milch, Fleisch, und Blut;
- Kombination mit Beta-Glucanase und/oder Mannanase für eine verbesserte Leistung an komplexen Anschmutzungen, die Stärke und/oder andere pflanzliche Polysaccharide enthalten, insbesondere solchen, die aus pflanzlichen Verdickungsmitteln resultieren, wie sie in industriell hergestellten Lebensmitteln eingesetzt werden;
- Kombination mit einem unlöslichen Builder, insbesondere Zeolith für eine verbesserte Leistung im Vergleich zu einem löslichem Builder;
- Kombination mit Zink-, Aluminium-, Calcium-, Magnesium- und/oder Wismutsalzen;
- Kombination mit Amylasen für verbesserte Leistung an Kakao, Eiweiß-Stärke-Anschmutzungen;
- Kombination mit sulfonsäuregruppenhaltigen Polymeren, hydrophob modifizierten Polymeren und/oder amphoteren oder kationisch modifizierten Polymeren zur Verbesserung des bei der maschinellen Geschirrreinigung insgesamt erzielbaren Ergebnisses (siehe oben);
- Kombination mit Cobuildern, insbesondere Methylglycin-Diessigsäure oder Methylglycin-Diacetat (MGDA), Glutaminsäurediessigsäure oder Glutaminsäurediacetat (GLDA), Asparaginsäurediessigsäure oder Asparaginsäurediacetat (ASDA), N-(2-Hydtoxyethyl)imido-Diessigsäure oder N-(2-Hydroxyethyl)imido-Diacetat (HEIDA), Iminodibemsteinsäure oder Iminodisuccinat (IDS), Hydroxy-Iminodibernsteinsäure oder Hydroxy-Iminodisuccinat (HIDS) und/oder Ethylendiamin-N,N'-dibernsteinsäure oder Ethylendiamin-N,N'-disuccinat (EDDS); und
- Kombination mit speziellen nichtionischen Tensiden wie den Hydroxymischethern, die unten genauer aufgeführt werden.

Besonders vorteilhafte Wirkungen dieser Lösungen ergeben sich also bei diesen Kombinationen, die somit entsprechend bevorzugt sind.

Besonders vorteilhafte Reinigungsleistungen zeigen solche erfindungsgemäßen maschinellen Geschirrspülmittel, bei denen das Enzymgranulat neben dem Enzym weiterhin auch ein anorganisches und/oder organisches Trägermaterial sowie als Granulierhilfsmittel eine phosphatierte Stärke umfasst.

Auch der Zusatz von hygroskopischen Polyolen zu den Enzymgranulaten und die Beschichtung der Enzymgranulate haben sich als für die Produkts- und Lagerstabilität vorteilhaft erwiesen und wird daher ebenfalls bevorzugt.

Die Verbesserung des Leistungsprofils wurde insbesondere bei solchen maschinellen Geschirrspülmitteln erreicht, die
- einen Gewichtsanteil des Enzymgranulats zwischen 0,2 und 7 Gew.-%, vorzugsweise 0,4 und 6 Gew.-% und insbesondere 0,6 und 5 Gew.-%, jeweils bezogen auf das Gesamtgewicht des maschinellen Geschirrspülmittels,
- einen Gewichtsanteil von Citrat/Citronensäure zwischen 5 und 60 Gew.-%, vorzugsweise 7 und 50 Gew.-% und insbesondere 10 bis 40 Gew.-%, jeweils bezogen auf das Gesamtgewicht des maschinellen Geschirrspülmittels
- einen Gewichtsanteil von Carbonat zwischen 10 und 60 Gew.-%, vorzugsweise 15 und 50 Gew.-% und insbesondere 20 bis 40 Gew.-%, jeweils bezogen auf das Gesamtgewicht des maschinellen Geschirrspülmittels
aufweisen. Entsprechende maschinelle Geschirrspülmittel sind daher erfindungsgemäß bevorzugt.

Die Bezeichnung "Citrat" umfasst die Salze der Citronensäure, insbesondere deren Akalisalze wie beispielsweise das Natriumcitrat.
Die Bezeichnung "Carbonat" umfasst die Salze der Kohlensäure, insbesondere die Carbonate und Hydrogencarbonate. Mit besonderem Vorzug werden Alkalicarbonate, insbesondere Natriumcarbonate und Natriumhydrogencarbonat sowie Kaliumcarbonat eingesetzt. Percarbonate wie das Natriumpercarbonat zählen nicht zur Gruppe der Carbonate.

Erfindungsgemäße Geschirrspülmittel, die neben einem erfindungsgemäßen Protease-haltigen Enzymgranulat weiterhin ein Citrat/Citronensäure/Carbonat-Buildersystem enthalten, eignen sich in besonderer Weise zur Beseitigung Einweiß-haltiger Anschmutzungen beim maschinellen Geschirrspülen, weshalb die Verwendung entsprechender Mittel zur Beseitigung Eiweiß-haltiger Verschmutzungen in einem maschinellen Geschirrspülverfahren ein weiterer Gegenstand der vorliegenden Anmeldung ist.

Die erfindungsgemäße Wirkung der o.g. Enzymgranulate kann außer durch den Zusatz von Citrat/Citronensäure/Carbonat-Buildersystemen weiterhin auch durch den Zusatz Sulfonsäuregruppen-haltiger, Polymere sowie durch eine chemische und/oder physikalische Stabilisierung der Enzymgranulate gesteigert werden. Als in Bezug auf ihre Stabilität in Produktion und Lagerung vorteilhaft haben sich Enzymgranulate erwiesen, die einen Aktivsubstanzgehalt von 5,5 bis 30 Gew.-% und insbesondere 6,0 bis 12 Gew.-% aufweisen. Der Einsatz entsprechender Enzymgranulate wird daher erfindungsgemäß bevorzugt.

Eine bevorzugte Ausführungsform ist ein erfindungsgemäßes Geschirrspülmittel, enthaltend als Enzymgranulat ein erfindungsgemäßes Protease-Granulat, wobei das Mittel einen Gehalt von 0,000001 bis 5, vorzugsweise von 0,00005 bis 0,1, besonders bevorzugt von 0,00001 bis 0,072 Gew.-% Enzymprotein dieser granulierten Protease, bezogen auf die Gesamtrezeptur des Geschirrspülmittels aufweist, wobei weiterhin ganz besonders bevorzugt für maschinelle Geschirrspülmittel 0,003 bis 0,13 Gew.-% Enzymprotein dieser granulierten Protease, bezogen auf die Gesamtrezeptur des Mittels, vorliegen.

Diese Werte beziehen sich auf die Gesamtrezeptur der betreffenden Mittel und haben sich hinsichtlich der Wasch- bzw. Reinigungsleistung dieser Mittel als vorteilhaft herausgestellt. Solche geeignet erscheinenden Konzentrationswerte können durch eine entsprechende Einwaage der efindungsgemäßen Enzymgranulate eingestellt werden. Da es sich dabei um Hochkonzentrate handelt, zeigt sich hierbei der Vorteil, dass im Vergleich zu niedriger konzentrierten Enzymgranulaten im Mittel mehr Restvolumen verbleibt, das für weitere Wirkstoffe genutzt werden kann.

Eine bevorzugte Ausführungsform ist ein efindungsgemäßes Geschirrspülmittel, enthaltend als zusätzliches Enzymgranulat ein erfindungsgemäßes α-Amylase- oder CGTase-Granulat, wobei das Mittel einen Gehalt von 0,000001 bis 5, vorzugsweise von 0,00005 bis 0,1, besonders bevorzugt von 0,00001 bis 0,072 Gew.-% Enzymprotein dieser granulierten α-Amylase- oder CGTase, bezogen auf die Gesamtrezeptur des Mittels aufweist.

Hierzu gilt das oben über die Protease-Konzentrationen Gesagte analog.

Eine bevorzugte Ausführungsform ist ein erfindungsgemäßes Geschirrspülmittel, enthaltend als zusätzliches Enzymgranulat ein erfindungsgemäßes Granulat einer Cellulase (Endoglucanase oder Cellobiohydrolase oder eine Mischung davon), β-Glucanase und/oder Mannanase, wobei das Mittel einen Gehalt von 0,000001 bis 5, vorzugsweise von 0,00005 bis 0,1, besonders bevorzugt von 0,00001 bis 0,072 Gew.-% Enzymprotein dieser granulierten Cellulase (Endoglucanase oder Cellobiohydrolase oder eine Mischung davon), β-Glucanase und/oder Mannanase, bezogen auf die Gesamtrezeptur des Mittels aufweist, wobei für β-Glucanase oder Mannanase weiterhin ganz besonders bevorzugt die Konzentration für maschinelle Geschirrspülmittel 0,001 bis 0,0072 Gew.-% und für Textilwaschmittel 0,0009 bis 0,04 Gew.-%, jeweils bezogen auf die Gesamtrezeptur des Mittels, beträgt und wobei für Cellulase weiterhin ganz besonders bevorzugt die Konzentration für Textilwaschmittel 0,0007 bis 0,005 Gew.-%, bezogen auf die Gesamtrezeptur des Mittels, beträgt.

Hierzu gilt das oben über die Protease-Konzentrationen Gesagte analog.

Es ist allgemein bekannt, dass die Leistung von Enzymen durch Bleichmittel ungünstig beeinflusst wird. Deshalb war es um so überraschender, dass festgestellt werden konnte, dass die erfindungsgemäßen Wasch- oder Reinigungsmittel, wenn sie Bleichmittel enthalten, eine signifikant verbesserte Leistung aufweisen. Überraschenderweise werden diese Effekte sowohl in festen als auch in flüssigen Reinigungsmitteln gefunden..

Eine bevorzugte Ausführungsform ist somit ein erfindungsgemäßes Wasch- oder Reinigungsmittel, enthaltend ein Bleichmittel oder ein Bleichmittelsystem aus mehreren Komponenten.

Eine hierunter bevorzugte Ausführungsform ist ein erfindungsgemäßes Wasch- oder Reinigungsmittel, wobei es sich bei dem Bleichmittel oder einem Bestandteil des Bleichmittelsystems um eine organische oder anorganische Perverbindung und/oder um ein oxidatives Enzym handelt, wobei vorzugsweise das oxidative Enzym in Form eines erfindungsgemäßen Granulats vorliegt.

Eine bevorzugte Ausführungsform ist ein erfindungsgemäßes Wasch- oder Reinigungsmittel, wobei es sich bei dem Bleichmittel oder dem Bestandteil des Bleichmittelsystems um Percarbonat, Perborat, Phthalimidoperoxyhexansäure (PAP) und/oder eine Oxidase handelt, wobei im letzten Fall vorzugsweise zusätzlich ein Substrat dieser Oxidase enthalten ist.

In einer bevorzugten Ausführungsform der Erfindung enthalten die Mittel Percarbonat, insbesondere eine Kombination aus Percarbonat und dem Bleichaktivator TAED. In einer weiteren bevorzugten Ausführungsform der Erfindung enthalten die Mittel Phthalimidoperoxyhexansäure (PAP), gegebenenfalls in Kombination mit TAED.

Bei maschinellen Geschirrspülmitteln wurde insbesondere hinsichtlich der Klarspülwirkung eine überraschend positive Wirkung bei der Kombination von Klarspülpolymeren (s.u.) beziehungsweise eines Klarspültensids mit einem erfindungsgemäß einzusetzenden Granulat festgestellt. Diese positive Wirkung zeigt sich insbesondere darin, dass die Zahl der Wasserflecken bei Anwendung eines erfindungsgemäßen maschinellen Geschirrspülmittels, enthaltend ein Klarspülpolymer beziehungsweise ein Klarspültensid, deutlich verringert wird.

Eine bevorzugte Ausführungsform ist ein erfindungsgemäßes Reinigungsmittel, wobei es sich bei dem Polymer um ein sulfonsäuregruppenhaltiges Polymer handelt.

Besonders bevorzugt als Sulfonsäuregruppen-haltige Polymere einsetzbar sind Copolymere aus ungesättigten Carbonsäuren, Sulfonsäuregruppen-haltigen Monomeren und gegebenenfalls weiteren ionogenen oder nichtionogenen Monomeren.

Eine bevorzugte Ausführungsform ist ein erfindungsgemäßes Wasch- oder Reinigungsmittel, wobei es sich bei dem Polymer um ein anionisches oder kationisches Polymer handelt.

Geeignete Beispiele hierfür sind Sulfopolymere und Polyacrylate, wie sie beispielsweise unter dem Handelsnamen Mirapol® von der Firma Rhodia erhältlich sind.

"Kationische Polymere" im Sinne der vorliegenden Erfindung sind Polymere, welche eine positive Ladung im Polymermolekül tragen. Diese kann beispielsweise durch in der Polymerkette vorliegende (Alkyl-)Ammoniumgruppierungen oder andere positiv geladene Gruppen realisiert werden. Besonders bevorzugte kationische Polymere stammen aus den Gruppen der quaternierten Cellulose-Derivate, der Polysiloxane mit quaternären Gruppen, der kationischen Guar-Derivate, der polymeren Dimethyldiallylammoniumsalze und deren Copolymere mit Estern und Amiden von Acrylsäure und Methacrylsäure, der Copolymere des Vinylpyrrolidons mit quaternierten Derivaten des Dialkylaminoacrylats und -methacrylats, der Vinylpyrrolidon-Methoimidazoliniumchlorid-Copolymere, der quaternierter Polyvinylalkohole oder der unter den INCI-Bezeichnungen Polyquaternium 2, Polyquaternium 17, Polyquaternium 18 und Polyquaternium 27 angegeben Polymere.

Eine hierunter bevorzugte Ausführungsform ist ein erfindungsgemäßes Reinigungsmittel, wobei es sich bei dem Polymer um ein Klarspülpolymer handelt.

Diese Polymere sind nicht eigentlich über ihre Zusammensetzung sondern über ihre Funktion definiert. Sie erleichtern bei Einsatz in Reinigern für feste Oberflächen, insbesondere in maschinellen Geschirrspülmitteln das Ablaufen des Wassers und wirken so der Bildung von Flecken entgegen, die auf eintrocknende Wassertropfen zurückzuführen sind.

Als Klarspülpolymere geeignete Sulfopolymere werden beispielsweise in der Anmeldung DE 10032612 A1 offenbart und sind bereits oben vorgestellt worden. Es handelt sich um Copolymere aus (i) ungesättigten Carbonsäuren, (ii) Sulfonsäuregruppen-haltigen Monomeren und (iii) optional weiteren ionischen oder nichtionogenen Monomeren. Solche Polymere werden im Rahmen von Geschirrspülmittelrezepturen und in Kombination mit einer speziellen Anmylase in der Anmeldung WO 2005/108540 A1 offenbart. In besonders bevorzugten Ausführungsformen der vorliegenden Erfindung handelt es sich somit um Mittel mit solchen Polymeren und erfindungsgemäß granulierten α-Amylasen, insbesondere den ebenfalls in WO 2005/108540 A1 offenbarten α-Amylasen.

Eine weiterhin bevorzugte Ausführungsform ist ein erfindungsgemäßes Reinigungsmittel, vorzugsweise mit einem Klarspülpolymer, wobei es sich bei dem Polymer um ein amphoteres oder kationisch modifiziertes Polymer handelt.

"Amphotere Polymere" im Sinne der vorliegenden Erfindung weisen neben einer positiv geladenen Gruppe in der Polymerkette weiterhin auch negativ geladenen Gruppen bzw. Monomereinheiten auf. Bei diesen Gruppen kann es sich beispielsweise um Carbonsäuren, Sulfonsäuren oder Phosphonsäuren handeln.

Insbesondere sind derartige Geschirrspülmittel bevorzugt, bei denen der Wassergehalt der festen Mittel beziehungsweise der festen Phase 1 bis 20 Gew.-%, vorzugsweise 3 bis 18 Gew.-% und insbesondere maximal 15 Gew.-% beträgt. Beispiele für besonders bevorzugte Wassergehalte der erfindungsgemäßen Mittel in fester Form oder der festen Phase, welche das erfindungsgemäße Granulat enthält, sind 5 Gew.-%, 10 Gew.-%, 12 Gew.-% oder 15 Gew.-%, wobei auch alle Zwischenwerte möglich sind.

Eine bevorzugte Ausführungsform ist ein erfindungsgemäßes festes Geschirrspülmittel, dadurch gekennzeichnet, dass die feste(n) Phase(n) in verpresster Form, vorzugsweise in Form eines tablettierten Formkörpers vorliegt/vorliegen.

Die Herstellung dieser Formkörper, die jeweils als einzelne Dosiereinheit angesehen werden, erfolgt vorzugsweise durch Tablettierung, Extrusion oder Walzenkompaktierung. Die Tablettierung erfolgt bevorzugt in dem Fachmann bekannter Weise durch Verpressung partikulärer Ausgangssubstanzen. Zur Herstellung der Tabletten wird das Vorgemisch in einer so genannten Matrize zwischen zwei Stempeln zu einem festen Komprimat verdichtet. Dieser Vorgang, der im folgenden kurz als Tablettierung bezeichnet wird, gliedert sich in vier Abschnitte: Dosierung, Verdichtung (elastische Verformung), plastische Verformung und Ausstoßen. Die Tablettierung erfolgt dabei vorzugsweise auf so genannten Rundläuferpressen.

Bei der Tablettierung mit Rundläuferpressen hat es sich als vorteilhaft erwiesen, die Tablettierung mit möglichst geringen Gewichtschwankungen der Tablette durchzuführen. Auf diese Weise lassen sich auch die Härteschwankungen der Tablette reduzieren. Geringe Gewichtschwankungen können auf folgende Weise erzielt werden. Zur Verminderung von Stempelanbackungen bieten sich sämtliche aus der Technik bekannte Antihaftbeschichtungen an. Besonders vorteilhaft sind Kunststoffbeschichtungen, Kunststoffeinlagen oder Kunststoffstempel. Auch drehende Stempel haben sich als vorteilhaft erwiesen, wobei nach Möglichkeit Ober- und Unterstempel drehbar ausgeführt sein sollten. Bei drehenden Stempeln kann auf eine Kunststoffeinlage in der Regel verzichtet werden. Hier sollten die Stempeloberflächen elektropoliert sein.

Die einzelnen Phasen der zwei- oder mehrphasigen Tablette sind vorzugsweise in Schichten angeordnet. Die Tablette kann zwei, drei, vier oder mehr Schichten aufweisen.

Vorzugsweise weist mindestens eine der Schichten eine Kavität auf. Der Begriff "Kavität" kennzeichnet im Rahmen der vorliegenden Erfindung Mulden, welche sich beispielsweise in der Boden- und/oder Dachfläche des Formkörpers befinden.

### Beispiele

### Beispiel 1

Leistungsvergleich erfindungsgemäßer Granulate mit niedriger konzentrierten Granulaten in einem maschinellen Geschirrspülmittel

Zum Vergleich erfindungsgemäßer Granulate mit solchen des Stands der Technik wurde zunächst folgende proteasefreie Rahmenrezeptur für ein festes maschinelles Geschirrspülmittel hergestellt:

| Inhaltsstoff | Gehalt [Gew.-%] |
|---|---|
| Na-Tripolyphosphat | 40 - 50 |
| Nichtionisches Tensid | 3 - 8 |
| Acrylsäure/Sulfonsäure-Copolymer | 5 - 10 |
| Acrylsäurepolymer | 5 - 10 |
| Na-percarbonat | 10 |
| Na-carbonat | 10 - 15 |
| Amylase-Granulat gemäß DE 102005062984.9 (nicht erfindungsgemäß) | 0,5 - 1,5 |
| Zinkacetat | 0,5 - 1 |
| Wasser, Salze, Farbstoffe | ad 100% |

Außerdem wurden zwei verschiedene Granulate der Alkalischen Protease F49 aus *Bacillus lentus* DSM 5483 gemäß WO 95/23221 A1 hergestellt. Die Herstellung erfolgte gemäß WO 97/40128 A1 und WO 98/26037 A2. Die Protease wurde zu 4,5 Gew.-% Aktivsubstanz (Stand der Technik) bzw. 6,9 Gew.-% Aktivsubstanz (Erfindung), bezogen auf das Granulat eingewogen. Der Gewichtsausgleich erfolgte über die komplette restliche Rezeptur für den Granulatkern (ohne Beschichtung).

Zur Herstellung der Granulate war eine Präparation dieser Protease mit 5.797 Protease-Einheiten (PE) pro mg eingesetzt worden, bestimmt nach der in Tenside, Band 7 (1970), Seite 125-132 beschriebenen Methode. 5.797 PE entsprechen ca. 3,4782 KNU (Kilo-Novo-Protease-Units). Die Einwaage von 45 mg bzw. 69 mg auf 1 g Granulat lieferte die genannten 4,5 bzw. 6,9 Gew.-% und Granulate mit ca. 260.869 bzw. ca. 400.000 PE pro g Granulat.

In den Gesamtrezepturen der vollformulierten Geschirrspülmittel, bestehend aus der oben genannten Rahmenrezeptur (in allen Ansätzen identisch) und den unterschiedlichen Proteasegranulaten, wurden die in der nachfolgenden Tabelle angegebenen Endkonzentrationen eingestellt. Der Gewichtsausgleich erfolgte hier über Na-Tripolyphosphat.

| Ansatz | Aktivsubstanz-Gehalt der Protease im Granulat [Gew.-%] | Konzentration des Granulats im Mittel [Gew.-%] | Konzentration der Protease im Mittel [Gew.-%] | Aktivität der Protease im Mittel [PE / g] |
|---|---|---|---|---|
| **1** | 4,5 | 4,5 | 0,2025 | 11.739 |
| **2** | 6,9 | 2,93 | 0,20217 | 11.720 |
| **3** | 6,9 | 4,5 | 0,3105 | 18.000 |

Aus den Rezepturen wurden in einer Tablettierungspresse der Firma Paul-Otto Weber Maschinen + Apparatebau GmbH unter einem Druck von 25 N/cm² Einphasen-Tabletten gepresst.

Gefäße mit harten, glatten Oberflächen wurden standardisiert mit einer Ei/Milch-Anschmutzung versehen und mit einer handelsüblichen Haushaltsgeschirrspülmaschine gespült, nämlich bei 50°C mit dem Normalprogramm der Geschirrspülmaschine des Typs Miele^{®} ECOSENSOR G 651 SC Plus-3. Pro Spülgang wurden jeweils 20 g des Spülmittels 1, 2 oder 3 verwendet. Die Wasserhärte betrug 21° deutscher Härte. Die Leistung wurde als Abtrag an Weicheiblechen gemäß SOFW-Journal 11/1998, S. 1022 bis 1034 bestimmt.

Nach dem Spülen wurde der Abtrag der Anschmutzungen gravimetrisch in Prozent ermittelt. Dafür wurde die Differenz aus dem Gewicht des verschmutzten und anschließend gespülten Gefäßes und dem Anfangsgewicht des Gefäßes in Relation zu der Gewichtsdifferenz des nicht gespülten Gefäßes zum Anfangsgewicht gesetzt. Diese Relation kann als Prozent Abtrag angesehen werden. Die erhaltenen Ergebnisse werden in folgender Tabelle zusammengestellt; dort sind die Mittelwerte aus jeweils 8 Messungen angegeben.

| Ansatz | Abtrag in % |
|---|---|
| **1** | 77 |
| **2** | 81,5 |
| **3** | 88 |

Durch Vergleich der beiden Ansätze 1 und 2, die sich lediglich hinsichtlich der Konzentration der Aktivsubstanz im Granulat, praktisch aber nicht in der Endkonzentration der Protease im vollformulierten Mittel unterscheiden, erkennt man folgendes: Allein die Erhöhung der Konzentration der Aktivsubstanz im Granulat führt zu einer Verbesserung der Reinigungsleistung, auch wenn die Endkonzentration nicht verändert (sondern sogar geringfügig verringert) wird. Wegen des Gleichbleibens der Endkonzentration wäre dieselbe Leistung zu erwarten gewesen.

Durch Vergleich der beiden Ansätze 2 und 3, die sich nicht in der Konzentration der Aktivsubstanz im Granulat, aber in der Endkonzentration der Protease im vollformulierten Mittel unterscheiden, erkennt man folgendes: Durch Erhöhung der Endkonzentration an Protease läßt sich die auf die Hochkonzentrierung der Granulate zurückzuführende Leistung noch weiter steigern.

### Beispiel 2

Zur Verdeutlichung der vorteilhaften Kombination erfindungsgemäßer Enzymgranulate mit Sulfonsäuregruppen-haltigen Polymeren wurden die folgenden Rezepturen für feste maschinelle Geschirrspülmittel hergestellt:

| Inhaltsstoff | E1 | V1 | V2 |
|---|---|---|---|
| | [Gew.-%] | [Gew.-%] | [Gew.-%] |
| Natriumcitrat | 25 | 37 | 25 |
| MGDA | 8 | 8 | 8 |
| HEDP | 2 | 2 | 2 |
| Natriumsilikat | 2 | 2 | 2 |
| Natriumcarbonat | 28 | 28 | 28 |
| Polyacrylat | 1 | 1 | 1 |
| Natriumpercarbonat | 10 | 10 | 10 |
| Bleichaktivator/-katalysator | 2,5 | 2,5 | 2,5 |
| Nichtionisches Tensid | 5 | 5 | 5 |
| Sulfonsäuregruppen-haltiges Polymer | 12 | -- | 12 |
| Proteasegranulat | 2,5 * | 2,5 * | 3,8 ** |
| Wasser, Salze, Farbstoffe | ad 100% | ad 100% | ad 100% |

| | | | |
|---|---|---|---|
| * Enzymgranulat mit einem Enzymgehalt von 6,9 Gew.-% an Aktivsubstanz des Enzyms ** Enzymgranulat mit einem Enzymgehalt von 4,5 Gew.-% an Aktivsubstanz des Enzyms | | | |

Die Rezepturen wurden zu Tabletten mit einem Gewicht von 20 g verpresst.
Gefäße mit harten, glatten Oberflächen wurden standardisiert mit einer Anschmutzung aus eingebranntem Hackfleisch versehen und in einer Geschirrspülmaschine (Bosch 57M22EU) in einem Standardreinigungsprogramm bei 40°C gereinigt. Pro Reinigungsgang wurde über die Dosiervorrichtung der Geschirrspülmaschine eine Tablette (20 g) der o.g. Zusammensetzung in die Reinigungsflotte dosiert. Die Bestimmung der Anschmutzung nach der Reinigung ergab das folgende Ergebnis:

| Versuch | E1 | V1 | V2 |
|---|---|---|---|
| Reinigungsleistung | 5,8 | 3,8 | 3,2 |

Bewertungsskala Reinigung: 10 = keine Verunreinigung bis 0 = starke Verunreinigung

### Beispiel 3

Zur Verdeutlichung der vorteilhaften Kombination erfindungsgemäßer Enzymgranulate mit Citrat/Citronensäure/Carbonat-Buildersystemen wurden die folgenden Rezepturen für feste maschinelle Geschirrspülmittel hergestellt:

| Inhaltsstoff | E1 | V1 |
|---|---|---|
| | [Gew.-%] | [Gew.-%] |
| Phosphat | -- | 37 |
| Natriumcitrat | 37 | -- |
| HEDP | 0,8 | 0,8 |
| Natriumsilikat | 2,0 | 2,0 |
| Natriumcarbonat | 40 | 40 |
| Natriumpercarbonat | 10 | 10 |
| Bleichaktivator/-katalysator | 3,3 | 3,3 |
| Nichtionisches Tensid | 2,0 | 2,0 |
| Proteasegranulat | 2,2 * | 2,2 * |
| Amylase | 0,5 | 0,5 |
| Wasser, Salze, Farbstoffe | ad 100% | ad 100% |

| | | |
|---|---|---|
| * Enzymgranulat mit einem Enzymgehalt von 6,9 Gew.-% an Aktivsubstanz des Enzyms | | |

Die Rezepturen wurden zu Tabletten mit einem Gewicht von 18 g verpresst.
Gefäße mit harten, glatten Oberflächen wurden standardisiert mit Anschmutzungen entsprechend der IKW-Methode zur Bestimmung der Reinigungsleistung von maschinellen Geschirrspülreinigern versehen und in einer Geschirrspülmaschine (Miele G698SC) in einem Standardreinigungsprogramm bei 50°C gereinigt. Pro Reinigungsgang wurde über die Dosiervorrichtung der Geschirrspülmaschine eine Tablette (18 g) der o.g. Zusammensetzung in die Reinigungsflotte dosiert. Die Bestimmung der Anschmutzung (Eigelb) nach der Reinigung ergab das folgende Ergebnis:

| Versuch | E1 | V1 |
|---|---|---|
| Reinigungsleistung | 9,0 | 8,0 |

Bewertungsskala Reinigung: 10 = keine Verunreinigung bis 0 = starke Verunreinigung

## Patentansprüche

1. Phosphatfreies maschinelles Geschirrspülmittel, enthaltend, bezogen auf das Gesamtgewicht des maschinellen Geschirrspülmittels,
a) 0,2 bis 7 Gew.-% Enzymgranulat mit einem Enzymgehalt von 5,5 bis 30 Gew.-% an Aktivsubstanz des Enzyms, wobei es sich bei dem Enzym um ein Subtilisin handelt, bei welchem es sich um ein Wildtypenzym oder um eine Subtilisin-Variante handelt, wobei es sich bei dem Wildtypenzym bzw. dem Ausgangsenzym der Variante um die alkalische Protease aus *Bacillus amyloliquefaciens* (BPN') handelt und wobei es sich bei dem Enzym um eine Variante handelt, die zu der Ausgangsprotease auf Aminosäureebene über die Gesamtlänge der Variante eine Sequenzidentität von mindestens 90 % aufweist;
b) zwischen 5 bis 60 Gew.-% Citrat/Citronensäure;
c) zwischen 10 bis 60 Gew.-% Carbonat.

2. Verwendung eines maschinellen Geschirrspülmittels nach Anspruch 1 zur Beseitigung Eiweiß-haltiger Verschmutzungen in einem maschinellen Geschirrspülverfahren.

3. Verfahren zur Reinigung von Geschirr in einer Geschirrspülmaschine unter Einsatz eines maschinellen Geschirrspülmittels nach Anspruch 1.

## Claims

1. Phosphate-free machine dishwashing detergent, comprising, based on the total weight of the machine dishwashing detergent,
a) 0.2 to 7 wt % enzyme granulate with an enzyme content of 5.5 to 30 wt % of active substance of the enzyme, wherein the enzyme concerns a subtilisin, in which a wild type enzyme or a subtilisin variant is concerned, wherein the wild type enzyme or the starting enzyme of the variant concerns the alkaline protease from *Bacillus amyloliquefaciens* (BPN') and wherein the enzyme concerns a variant that at the amino acid level possesses a sequence identity of at least 90 % to the starting enzyme over the total length of the variant;
b) between 5 to 60 wt % citrate/citric acid;
c) between 10 to 60 wt % carbonate.

2. Use of a machine dishwashing detergent according to claim 1 for eliminating protein-containing stains in a machine dishwashing process.

3. Process for cleaning tableware in a dishwasher by using a machine dishwashing detergent according to claim 1.

## Revendications

1. Détergent sans phosphates pour lave-vaisselle, contenant, par rapport au poids total dudit détergent pour lave-vaisselle,
a) 0,2 à 7 % en poids de granulés d'enzyme ayant une teneur en enzyme comprise entre 5,5 et 30 % en poids, exprimée en substance active de l'enzyme, ladite enzyme étant une subtilisine correspondant à une enzyme de type sauvage ou à une variante de la subtilisine, l'enzyme de type sauvage ou l'enzyme de base de ladite variante étant la protéase alcaline issue de *Bacillus amyloliquefaciens* (BPN') et ladite enzyme étant une variante laquelle présente, sur l'intégralité de la longueur de ladite variante, une identité de séquence en termes d'acides aminés d'au moins 90 % par rapport à la protéase de base ;
b) entre 5 et 60 % en poids de citrate / d'acide citrique ;
c) entre 10 et 60 % en poids de carbonate.

2. Utilisation d'un détergent pour lave-vaisselle selon la revendication 1 pour éliminer des salissures protéiques dans un procédé de lavage de vaisselle en machine.

3. Procédé destiné à nettoyer de la vaisselle dans un lave-vaisselle en mettant en oeuvre un détergent pour lave-vaisselle selon la revendication 1.
